# FASCICULE DE BREVET EUROPEEN

(11) **EP 3 801 661 B1**
(45) Date de publication et mention de la délivrance du brevet: **06.08.2025**
(21) Numéro de dépôt: 19737847.4
(22) Date de dépôt: 05.06.2019
(51) Int. Cl.: A61L 31/02, A61L 31/10

(54) **ENDOPROTHESE VASCULAIRE A PROPRIETES ANTI-THROMBOTIQUES**
VASKULÄRER STENT MIT ANTITHROMBOTISCHEN EIGENSCHAFTEN
VASCULAR STENT WITH ANTITHROMBOTIC PROPERTIES

(30) Priorité: 08.06.2018 FR 1854996
(43) Date de publication de la demande: 14.04.2021
(73) Titulaire: Université Paul Sabatier Toulouse III, 31400 Toulouse (FR); Centre Hospitalier Universitaire De Toulouse, 31300 Toulouse (FR); Centre national de la recherche scientifique, 75016 Paris (FR); Institut National de la Santé et de la Recherche Médicale (INSERM), 75013 Paris (FR)
(72) Inventeur: COGNARD, Christophe, 31200 TOULOUSE (FR); EICHWALD, Olivier, 31650 LAUZERVILLE (FR); GARCIA, Cédric, 31670 LABEGE (FR); MERBAHI, Nofel, 31400 TOULOUSE (FR); PAYRASTRE, Bernard, 31170 TOURNEFEUILLE (FR); SIE, Pierre, 31200 TOULOUSE (FR); TOKARSKI, Aurélie, 31500 TOULOUSE (FR); VUKASINOVIC, Ivan, BELGRADE 11000 (RS); YOUSFI, Mohammed, 31650 SAINT-ORENS-DE-GAMEVILLE (FR)
(74) Mandataire: Novagraaf Technologies
(86) Numéro de dépôt international: PCT/FR2019/051336
(87) Numéro de publication internationale: WO 2019/234350

(56) Documents cités:
- WO-A1-03/045582
- WO-A1-2017/004598
- WO-A2-95/29647
- US-A- 4 283 469

## Description

### Domaine technique

La présente invention appartient au domaine des dispositifs médicaux et plus particulièrement à celui des endoprothèses vasculaires.

L'invention se rapporte à une endoprothèse vasculaire, déployée ou non-déployée, dont la surface est recouverte par un film comprenant au moins une protéine, à un procédé de recouvrement de la surface d'une endoprothèse vasculaire par un film comprenant au moins une protéine et à un dispositif de mise en œuvre du procédé selon l'invention.

### État de la technique

Une endoprothèse vasculaire (appelé également « stent »,) est un dispositif le plus souvent métallique, maillé et tubulaire qui peut être positionné dans une cavité humaine ou animale pour dilater une sténose ou occlusion et maintenir la cavité ouverte. Les endoprothèses vasculaires sont essentiellement utilisées dans des artères, plus rarement dans les veines.

Les endoprothèses sont essentiellement utilisées en pathologies vasculaires (cardiologie et neurochirurgie endovasculaire) mais peuvent aussi être utilisées dans l'urètre, la trachée, l'œsophage ou bien les canaux biliaires.

L'endoprothèse vasculaire étant un matériel étranger au corps humain ou animal dans lequel elle est introduite, c'est un dispositif propice à la formation d'un caillot ou thrombus. Lorsqu'une endoprothèse vasculaire est mise en place, par exemple dans une artère, cette étape doit être suivie d'un traitement médicamenteux destiné à empêcher l'apparition de caillots. Ce traitement est indispensable, pendant au moins plusieurs semaines après la mise en place de l'endoprothèse vasculaire, jusqu'à ce qu'elle soit naturellement recouverte par les cellules de la paroi interne de l'artère. On a appelé ce processus « endothélialisation ».

Historiquement, le traitement médicamenteux est à base d'aspirine à faibles doses, associé à la ticlopidine (CAS n°55142-85-3). Aujourd'hui, ce traitement a évolué et l'association médicamenteuse comporte de l'aspirine et un autre antiagrégant plaquettaire tel que par exemple le clopidogrel (CAS n°113665-84-2), le prasugrel (CAS n°150322-43-3) ou bien encore le ticagrelor (CAS n°274693-27-5).

On distingue principalement deux types d'endoprothèses vasculaires ou « stents » : les stents dits « nus » et les stents dits « actifs » ou pharmaco-actifs. Les stents actifs sont également appelés endoprothèses artérielles à élution médicamenteuse (ou « drug-eluting stent » en anglais).

Les stents actifs se distinguent généralement des stents nus par l'intégration dans un polymère biodégradable plaqué sur le maillage métallique d'une substance antiproliférative qui diminue le risque de resténose différée, due à l'épaississement de la paroi vasculaire, en bloquant la prolifération des cellules musculaires lisses de la paroi du vaisseau. Ce phénomène est particulièrement critique en pathologie coronarienne car le vaisseau traité par le stent est fortement remanié par une lésion d'athérosclérose et très inflammatoire. Malheureusement, les substances diffusées par le stent actif, qui sont des antimitotiques, retardent le processus d'endothélialisation, qui repose sur la multiplication des progéniteurs endothéliaux circulants chargés de recouvrir le stent. Ceci laisse le stent actif et la paroi du vaisseau à son contact partiellement découverts ce qui induit un risque important de thrombose au niveau de la zone traitée. L'endothélialisation des mailles du stent est ainsi incomplète et retardée et la formation de caillots reste un danger potentiel justifiant un traitement antiagrégant plaquettaire puissant pendant une période prolongée de 3 à 12 mois suivant les cas en pathologie coronaire. Les stents actifs ne sont donc pas une solution idéale dans la mesure où ils prolongent la durée de la période à risque de thrombose et imposent un traitement à haut risque hémorragique systémique pendant une période longue.

Par ailleurs les stents actifs ne peuvent pas être utilisés en neurochirurgie dans le cas d'un AVC ischémique ou d'une rupture d'anévrisme, car ils retarderaient la cicatrisation du vaisseau que l'on vient de traiter.

Il existe donc un réel besoin d'endoprothèses vasculaires palliant ces défauts, inconvénients et obstacles de l'art antérieur, en empêchant l'activation de l'hémostase, en particulier des plaquettes au contact de l'endoprothèse sans affecter l'adhésion des progéniteurs endothéliaux circulants qui en se différenciant et se multipliant conduiront à la restauration de la couverture endothéliale luminale en contact avec le sang.

WO 95/29647 A2 décrit la préparation d'un stent recouvert d'un film de collagène ou d'albumine par electrodéposition.

### Exposé de l'invention

Il est du mérite des demanderesses d'avoir développé un nouveau type d'endoprothèse vasculaire, palliant à la fois les défauts et inconvénients précédemment cités des endoprothèses vasculaires nues ou actives (stents nus ou stents actifs).

Les endoprothèses vasculaires selon l'invention présentent ainsi plusieurs avantages, par rapport aux endoprothèses vasculaires connues dans l'état de la technique et possèdent notamment des propriétés antithrombotiques, sans avoir l'inconvénient de limiter le processus d'endothélialisation, même de manière partielle. Une fois endothélialisés, la paroi et l'endoprothèse deviennent peu thrombogènes et le traitement antiagrégant plaquettaire peut être allégé, avec des effets secondaires moindres.

Les endoprothèses vasculaires selon l'invention cumulent ainsi les avantages des stents nus et des stents actifs, sans en avoir les inconvénients.

Les endoprothèses vasculaires selon l'invention peuvent en outre posséder des propriétés antibactériennes et/ou anti-inflammatoires.

L'invention se rapporte à une endoprothèse vasculaire, déployée ou non-déployée, dont la surface est recouverte par un film comprenant au moins une protéine d'intérêt ayant été soumise à un champ électrique présentant une structure périodique.

Dans le cadre de l'invention, on entend par « protéines » ou « protéines d'intérêts », les protéines choisies dans le groupe comprenant une ou plusieurs protéines du plasma sanguin (telles que par exemple l'albumine, les immunoglobulines (anticorps, essentiellement des IgG), le fibrinogène, l'alpha-1-antitrypsine, l'alpha-2 macroglobuline, la transferrine, les lipoprotéines (essentiellement HDL et LDL)) et/ou une ou des macromolécules biologiques de synthèse permettant, après l'application d'un champ électrique pulsé, d'obtenir un film d'au moins une protéine fortement adhéré à la surface de l'endoprothèse et présentant des propriétés antithrombotiques sans empêcher l'endothélialisation. De préférence, la au moins une protéine d'intérêt peut être de l'albumine ou un mélange de protéines du plasma sanguin, comprenant de l'albumine.

Avantageusement, la au moins une protéine d'intérêt est soluble à un pH compris entre 3 et 12.

Le film comprenant la au moins une protéine d'intérêt selon l'invention, est un film de protéine(s) (par exemple plasmatiques) pouvant être déposé par l'application d'un champ électrique pulsé sur une solution comprenant au moins une protéine d'intérêt modifiée (par exemple déstructurée) ou non par l'application du champ électrique. Contrairement à une méthode de déposition classique, sans application d'un champ électrique, telle que par exemple décrite dans WO2017/004598, la au moins une protéine d'intérêt ayant été soumise au champ électrique recouvre l'endoprothèse de façon durable et avec des propriétés antithrombotiques démontrées (voir exemples ci-dessous).

Avantageusement, le film comprenant au moins une protéine d'intérêt peut être réparti de manière homogène sur la surface de l'endoprothèse vasculaire. Le film comprenant au moins une protéine d'intérêt a une épaisseur qui peut varier en fonction de la nature de la protéine, de la durée d'exposition au champ électrique.

L'épaisseur du film comprenant la au moins une protéine d'intérêt peut être supérieure ou égale à 30 Angströms, et est de préférence homogène sur la surface de l'endoprothèse. La concentration en protéine sur la surface de l'endoprothèse vasculaire peut être supérieure ou égale à 2 µg.cm⁻².

Avantageusement, l'endoprothèse vasculaire (ou stent) selon l'invention peut être toute endoprothèse vasculaire nue (ou stent nu) disponible dans le commerce que l'on a ensuite traitée pour former le film d'au moins une protéine d'intérêt. Il s'agit d'un dispositif médical, le plus souvent métallique, maillé et tubulaire, destiné à être inséré dans une cavité naturelle humaine ou animale pour la maintenir ouverte. L'endoprothèse vasculaire peut être constituée de matériaux divers tels que par exemple alliages métalliques, silicone et polymères. De préférence, l'endoprothèse vasculaire selon l'invention est métallique et comprend un ou plusieurs alliage(s) métallique(s). Par exemple, le ou les alliage(s) métallique(s) peuvent être choisis dans le groupe comprenant les alliages de type acier inoxydable, nickel/titane (tel que par exemple le nitinol), tantale, cobalt/chrome, platine/chrome, les alliages comprenant éventuellement du magnésium, et leurs mélanges.

Avantageusement, l'endoprothèse vasculaire selon l'invention peut être déployée ou non-déployée. Généralement, l'endoprothèse est non-déployée lorsqu'elle est conservée dans le micro-cathéter d'origine (dans lequel elle est commercialisée), avant implantation. On dit que l'endoprothèse est déployée lorsqu'une partie ou la totalité n'est plus dans le micro-cathéter qui sert à la mettre en place et que le maillage la constituant se déploie partiellement ou totalement en dehors du micro-cathéter d'origine. Lorsque qu'une endoprothèse vasculaire est déployée entièrement à l'air libre (sans contraintes), elle peut avoir un diamètre allant de 2 mm à 60 mm selon le type d'endoprothèse, par exemple de 2 mm à 10 mm pour un stent carotidien, de 2 mm à 5 mm pour un stent intracrânien, de 18 mm à 46 mm pour une endoprothèse vasculaire aortique. Une endoprothèse vasculaire peut avoir une longueur allant de 10 mm à 200 mm, par exemple de 10 mm à 70 mm pour un stent carotidien, de 10 mm à 35 mm pour un stent intracrânien ou jusqu'à 170 mm dans le cas de l'endoprothèse vasculaire aortique.

Avantageusement, l'endoprothèse vasculaire selon l'invention peut être :
- un stent cardiaque (souvent plus rigide qu'un stent intracrânien), nu ou comprenant par exemple un porteur biodégradable tel que de l'acide salicylique, de l'acide polylactique (PLLA) ou du magnésium. L'endoprothèse vasculaire selon l'invention peut comprendre en outre un revêtement de polymère contenant un médicament et/ou de différents matériaux (tels que des anticorps, du carbone, etc.),
- un stent cardiaque auto-expansible ou sur un ballon expansible (prémonté sur un ballon),
- un stent intracrânien nu à maillage lâche ou dense (Flow Diverter),
- un stent intracrânien, carotidien ou périphérique auto-expansible.

L'endoprothèse vasculaire peut par exemple être un stent destiné à être placé dans le système veineux (alliage nitinol ; diamètre 10 - 20 mm ; longueur 40 - 160 mm) ou un implant tel qu'un filtre cave (alliage nitinol, acier inoxydable ou cobalt-chrome ; diamètre 25 - 40 mm ; longueur jusqu'à 50 mm).

Les propriétés antithrombotiques des endoprothèses vasculaires selon l'invention peuvent être observées pendant une durée prolongée, allant d'une semaine à plusieurs mois. Par exemple, les propriétés antithrombotiques peuvent être observées pendant une durée supérieure ou égale à 3 mois, supérieure ou égale à 6 mois, voire même supérieure ou égale à 9 ou 12 mois.

L'invention se rapporte également à un procédé de recouvrement de la surface d'une endoprothèse vasculaire, déployée ou non-déployée, par un film d'au moins une protéine d'intérêt, comprenant les étapes de :
- mise en contact de l'endoprothèse vasculaire et d'une solution aqueuse comprenant au moins une protéine d'intérêt,
- application d'un champ électrique, présentant une structure périodique, généré par un système d'électrodes comprenant de préférence au moins une première électrode, un diélectrique et au moins une seconde électrode, ledit diélectrique isolant électriquement l'endoprothèse et la au moins une seconde électrode de la au moins une première électrode, et
- recouvrement de la surface de l'endoprothèse par le film d'au moins une protéine d'intérêt.

La au moins une première électrode est isolée de l'endoprothèse par un diélectrique. Le diélectrique isole la au moins une première électrode de l'endoprothèse vasculaire et de la au moins une seconde électrode. Il n'y a pas de courant électrique qui circule entre les électrodes du fait de la présence du diélectrique (isolant). Il en résulte la génération d'un champ électrique pulsé qui va entrainer le processus de formation du film d'au moins une protéine d'intérêt.

Avantageusement, lors de la mise en œuvre du procédé selon l'invention, l'endoprothèse vasculaire peut être déployée (ou partiellement déployée, une partie de l'endoprothèse restant enchâssée dans la gaine d'origine ou le micro cathéter servant à la mettre en place). Sous forme déployée, la surface est plus accessible et facilite le placement de la première électrode.

Avantageusement, la solution aqueuse comprend une ou plusieurs protéines d'intérêt telles que définies ci-dessus. La concentration en protéine(s) dans la solution aqueuse peut être supérieure ou égale à 0,1 mg/ml. Par exemple, lorsque la protéine est l'albumine, la concentration peut être comprise entre 5 et 50 mg/ml lorsque la solution aqueuse est une solution tamponnée comprenant du tampon phosphate salin (PBS) ou bien entre 30 et 50 mg/ml lorsque la solution aqueuse est du plasma sanguin. Le procédé selon l'invention peut être mis en œuvre dans une solution comprenant un ou plusieurs types de protéines. La solution aqueuse comprenant au moins une protéine peut être par exemple du plasma sanguin, une solution tamponnée comprenant un tampon tel que par exemple le PBS (tampon phosphate salin) ou de l'eau distillée salée (NaCl). La solution aqueuse peut ainsi être toute solution aqueuse ne dégradant pas les protéines d'intérêts qui y sont dissoutes.

Avantageusement, la solution aqueuse comprenant au moins une protéine d'intérêt peut être du plasma sanguin animal ou humain extrait directement du patient (transplant autologue). Cette solution offre l'avantage de traiter l'endoprothèse vasculaire juste avant son implantation avec un plasma limitant fortement les risques de rejets du patient.

Avantageusement, le pH de la solution aqueuse comprenant au moins une protéine d'intérêt a une valeur telle que la au moins une protéine est soluble durant la mise en œuvre du procédé. La solution aqueuse est de préférence homogène. Le pH de la solution aqueuse peut ainsi être compris entre 3 et 12, de préférence entre 6 et 9 et de manière encore plus préférée entre 7 et 8. Le pH de la solution aqueuse est par exemple 7,4.

On entend par « point isoélectrique » (PI) d'une protéine le pH auquel la charge électrique globale de la protéine est neutre. A son PI, la protéine possède un nombre égal de charges positives et de charges négatives et il n'y a pas de transport de protéines sous champ électrique. Si le pH est supérieur au PI alors la charge globale de la protéine est négative, dans le cas contraire, elle est positive. Par exemple, le pH du sang humain est régulé autour de 7,4 et l'albumine ayant un PI de 4,8, la charge totale de l'albumine dans le sang humain est négative (7,4 > 4,8). De préférence, dans le procédé selon l'invention, le pH de la solution aqueuse peut être supérieur ou bien inférieur au PI de la ou les protéine(s) formant le film d'au moins une protéine d'intérêt.

Lors de la mise en œuvre du procédé selon l'invention, la solution aqueuse comprenant au moins une protéine d'intérêt peut être mobile ou statique, de préférence statique, par rapport à l'endoprothèse vasculaire. De préférence, l'endoprothèse est immergée dans la solution comprenant au moins une protéine d'intérêt, de sorte que toute sa surface puisse être recouverte par le film de protéine lors de la mise en œuvre du procédé.

Avantageusement, le champ électrique est généré par un signal de tension appliqué au système d'électrodes. Le champ électrique peut ainsi être généré en mettant sous tension la au moins une première électrode, isolée par le diélectrique. L'amplitude du signal de tension peut être comprise dans un intervalle allant de 1 V à 50 kV. De préférence, l'amplitude est comprise dans un intervalle allant de 5 kV à 40 kV, et de manière encore plus préférée de 10 kV à 30 kV. La tension appliquée peut être positive et/ou négative, de préférence positive.

Le signal de tension (et donc du champ électrique résultant) présente une structure périodique. Il peut être caractérisé par sa période Tp (durée du motif qui se répète) et au sein d'une période par la durée Tv où la tension est non nulle. Le rapport Tv/Tp définit le rapport cyclique du signal périodique. Le motif qui se répète sur chaque période peut être continu par morceau (une impulsion par exemple), sinusoïdale, triangulaire ou en dents de scie. Par exemple, le signal de tension est une impulsion d'une durée Tv de 5.10⁻⁷ s qui se répète toutes les 0,01 s (il s'agit d'un signal périodique continu par morceau de fréquence 100 Hz et de rapport cyclique 5.10⁻⁵). La fréquence du signal peut ainsi avoir une valeur comprise dans un intervalle allant de 0,1 Hz à 100 kHz, de préférence de 1 Hz à 1 kHz, et le rapport cyclique une valeur comprise dans un intervalle allant de 5.10⁻⁸ à 1 (1 correspond à un signal alternatif redressé ou non), de préférence de 5.10⁻⁶ à 5.10⁻³.

Avantageusement, le champ électrique peut être généré pendant une durée supérieure ou égale à 10 secondes, de préférence supérieure ou égale à 5 minutes et de manière encore plus préférée supérieure ou égale à 10 ou 20 minutes. La durée peut être amenée à être augmentée ou diminuée en fonction du type de protéine, de la concentration en protéine dans la solution aqueuse et/ou du type d'endoprothèse vasculaire (par exemple le matériau).

Avantageusement, la tension est appliquée à la au moins une première électrode, la au moins une seconde électrode étant reliée à la masse. La tension peut aussi être appliquée à la au moins une seconde électrode, la au moins une première électrode étant reliée à la masse. L'endoprothèse peut aussi servir d'électrode si elle est métallique et séparée de la seconde électrode par un diélectrique.

Avantageusement, le procédé selon l'invention peut être réalisé en conditions stériles ou non stériles. Le procédé peut comprendre en outre une étape de stérilisation. Selon l'invention, toute méthode de stérilisation adaptée à la stérilisation d'endoprothèse vasculaire peut être mise en œuvre. L'étape de stérilisation n'altère pas les propriétés antithrombotiques de l'endoprothèse vasculaire selon l'invention.

Avantageusement, lors de la mise en œuvre du procédé selon l'invention, l'endoprothèse est positionnée dans un élément d'accueil, de préférence de forme tubulaire. Le procédé selon l'invention peut comprendre en outre une étape d'héparinisation dudit élément d'accueil.

L'invention se rapporte en outre à une endoprothèse vasculaire susceptible d'être obtenue par le procédé selon l'invention.

L'invention se rapport en outre à un dispositif de mise en œuvre du procédé selon l'invention comprenant :
- au moins une première électrode,
- au moins une seconde électrode,
- au moins un diélectrique isolant la première électrode de la seconde électrode,
- un élément d'accueil susceptible de contenir l'endoprothèse vasculaire, ledit élément d'accueil ayant une forme adaptée à la forme de l'endoprothèse vasculaire, ledit élément d'accueil étant identique ou différent du diélectrique, de préférence sous forme d'un tube diélectrique (par exemple en PVC) et,
- une source d'énergie électrique connectée aux électrodes et un régulateur de tension permettant de réguler la tension sur la au moins une première électrode et la au moins une seconde électrode afin de générer un champ électrique présentant une structure périodique.

Avantageusement, le dispositif pour la mise en œuvre du procédé de l'invention comprend un élément d'accueil de forme tubulaire destiné à recevoir l'endoprothèse vasculaire, de préférence cylindrique, comprenant une entrée pour la solution aqueuse comprenant au moins une protéine et une sortie, ledit dispositif étant muni d'un système d'électrodes comprenant au moins une première électrode, un diélectrique et au moins une seconde électrode ainsi qu'une source d'énergie électrique connectée aux électrodes et un régulateur de tension permettant de réguler la tension sur la au moins une première électrode et la au moins une seconde électrode afin de générer le champs électrique, ladite au moins une première électrode étant isolé de l'intérieur de l'élément d'accueil par ledit diélectrique.

Avantageusement, la au moins une première électrode est recouverte par le diélectrique. Dans le cadre de l'invention, on entend par diélectrique une substance ou un matériau ne possédant pas d'électrons libres capables de transporter un courant électrique, mais qui peut être polarisé par un champ électrique. Le diélectrique est positionné de manière à isoler la au moins une première électrode de l'endoprothèse vasculaire et de la seconde électrode, permettant ainsi, lors de l'application de la tension dans la première électrode de générer un champ électrique (la seconde électrode étant reliée à la masse). Le diélectrique peut être constitué de toute matière isolante. Par exemple, le diélectrique peut être en PVC, en parylène, en verre, en téflon, en céramique, en résine époxy, en Kapton ou en polyméthacrylate. Le diélectrique peut dans certains cas être le micro-cathéter d'origine dans lequel l'endoprothèse vasculaire est conservée avant son implantation. Le diélectrique peut avoir une épaisseur allant de 10 µm à 10 mm. Lorsque l'endoprothèse est positionnée dans le dispositif, on entend par épaisseur du diélectrique la distance qui sépare la partie métallique de l'électrode et l'endoprothèse, les différents éléments étant au contact.

Avantageusement, la tension permettant de générer un champ électrique peut aussi être appliquée dans la seconde électrode, la première électrode étant reliée, dans cette configuration, à la masse. L'endoprothèse, si elle est métallique, peut également servir d'électrode.

Avantageusement, l'élément d'accueil peut être tout support permettant le maintien de l'endoprothèse vasculaire pendant la mise en œuvre de procédé et en particulier, le micro-cathéter d'origine. Il est de forme tubulaire et il peut s'agir par exemple d'un tube en PVC (ou d'un des quelconques diélectriques cités en exemple ci-dessus) dans lequel l'endoprothèse vasculaire est introduite.

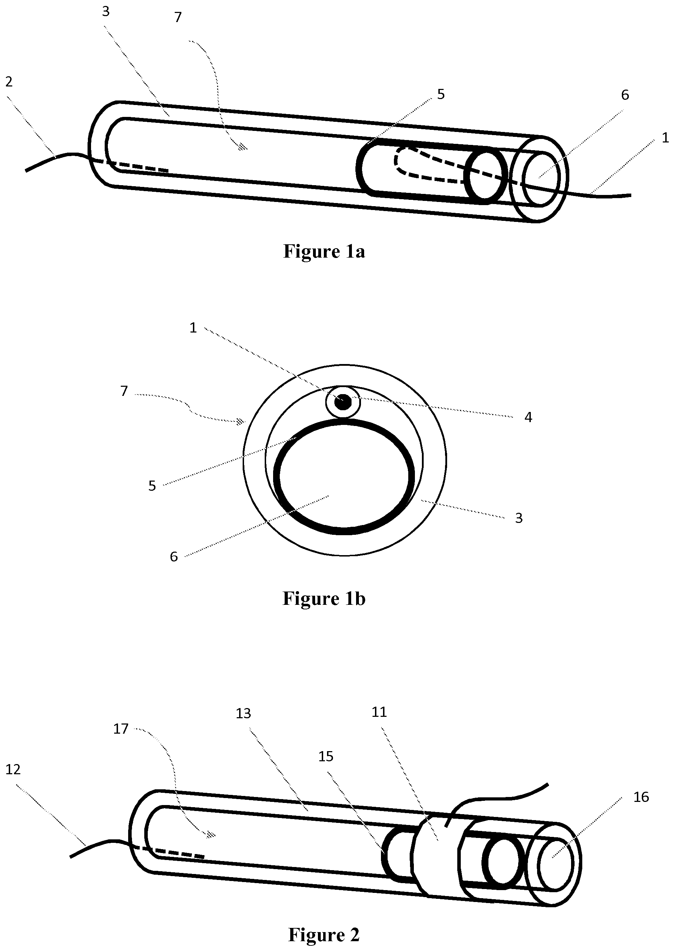

Les figures 1a et 1b représentent un dispositif 7 selon l'invention dans lequel une endoprothèse déployée 5 est positionnée. La première électrode 1 recouverte par le diélectrique 4 est positionnée entre l'élément d'accueil 3 (pouvant être isolant) et l'endoprothèse 5 (voir figure 1b en coupe). La seconde électrode 2 se situe à l'intérieur de l'élément d'accueil 3 à une distance de l'endoprothèse comprise dans un intervalle allant de 5 à 15 cm, de préférence 8 à 12 cm et de manière encore plus préférée 10 cm. La solution aqueuse comprenant au moins une protéine est destinée à circuler dans la cavité 6 de l'élément d'accueil 3. La génération du champ électrique pulsé entre les deux électrodes conduit à la formation du film d'au moins une protéine sur la surface de l'endoprothèse qui est en contact direct avec la solution aqueuse.

Avantageusement, l'élément d'accueil et le diélectrique peuvent être identiques ou différents Par exemple, le diélectrique peut être un tube en PVC dans lequel l'endoprothèse vasculaire sera placée lors de la mise en œuvre du procédé (voir figure 2). Dans cette configuration, l'épaisseur du diélectrique correspond à l'épaisseur de l'élément d'accueil qui peut être un tube en PVC (ou d'un des quelconques diélectriques cités en exemple ci-dessus).

La figure 2 représente un dispositif 17 selon l'invention dans lequel une endoprothèse 15, déployée ou non-déployée, est positionnée. La première électrode 11 se situe sur la surface extérieure de l'élément d'accueil 13 qui joue le rôle de diélectrique dans cette configuration. La seconde électrode 12 se situe à l'intérieur de l'élément d'accueil 13 à une distance de l'endoprothèse comprise dans un intervalle allant de 5 à 15 cm, de préférence 8 à 12 cm et de manière encore plus préférée de 8 cm. La solution aqueuse comprenant au moins une protéine est destinée à circuler dans la cavité 16 de l'élément d'accueil 13. La génération du champ électrique pulsé entre les deux électrodes conduit à la formation du film d'au moins une protéine sur la surface de l'endoprothèse qui est en contact avec la solution aqueuse. Dans cette configuration, la première électrode peut être une électrode dite annulaire ou bien une pluralité d'électrodes positionnées sur la surface externe de l'élément d'accueil. La seconde électrode peut être un simple fil électrique relié à la masse ou bien une électrode annulaire positionnée à l'intérieur ou à l'extérieur de l'élément d'accueil 13.

Avantageusement, l'élément d'accueil peut être le micro-cathéter d'origine de l'endoprothèse. Dans cet exemple, le procédé est mis-en-œuvre sur une endoprothèse non-déployée.

Avantageusement, lorsque l'endoprothèse possède intrinsèquement, en partie ou totalement, des propriétés diélectriques ou bien qu'elle comprend un ou plusieurs matériau(s) diélectrique(s), le procédé selon l'invention peut être mis en œuvre directement en positionnant le système d'électrode sur l'endoprothèse vasculaire. L'endoprothèse vasculaire est mise en contact avec la solution aqueuse comprenant au moins une protéine. Dans cette configuration, l'endoprothèse vasculaire joue le rôle du diélectrique. Le champ électrique appliqué par le système d'électrode est rendu possible par les propriétés isolantes de l'endoprothèse vasculaire.

Avantageusement, lorsque l'endoprothèse est métallique, elle peut servir d'électrode reliée à la masse ou mise sous tension. Dans cette configuration, l'électrode 2 ou 12 peut ne plus être nécessaire.

Avantageusement, les extrémités de l'élément d'accueil peuvent être fermées lors de la mise en œuvre du procédé, la solution aqueuse comprenant au moins une protéine est alors immobile ou stagnante durant la mise en œuvre du procédé.

L'invention se rapporte également à un kit comprenant un dispositif selon l'invention, une solution aqueuse comprenant au moins une protéine telle que définie ci-dessus et éventuellement une endoprothèse vasculaire.

L'invention se rapporte encore à une utilisation du dispositif ou du kit selon l'invention pour le recouvrement d'endoprothèses vasculaires par un film d'au moins une protéine d'intérêt.

D'autres avantages pourront encore apparaître à l'homme du métier à la lecture des exemples ci-dessous, illustrés par les figures annexées, donnés à titre illustratif et non limitatif.

### Brève description des figures

- Les figures 1a et 1b représentent un dispositif selon l'invention dans lequel l'élément d'accueil et le diélectrique sont différents et comprenant une endoprothèse vasculaire.
- La figure 2 représente un dispositif selon l'invention dans lequel l'élément d'accueil et le diélectrique sont identiques et comprenant une endoprothèse vasculaire.

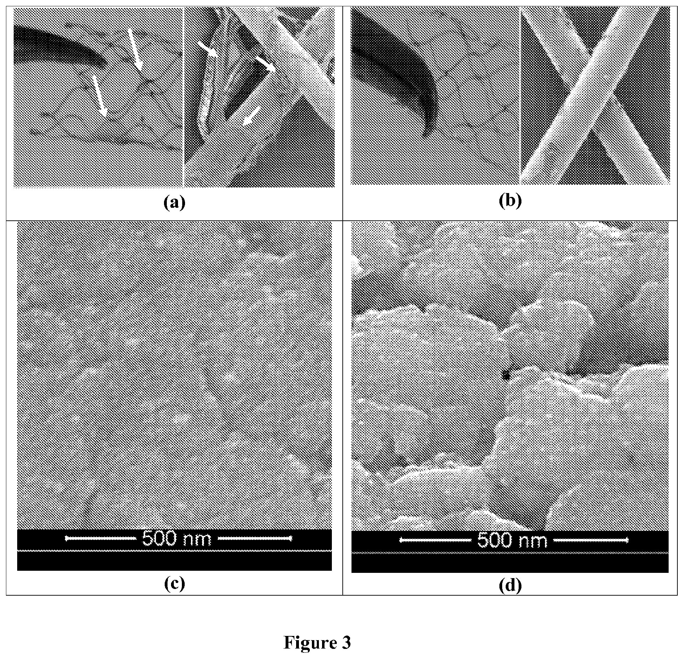

- La figure 3 représente une comparaison de l'aspect visuel et en microscopie électronique à balayage (MEB) de (a) l'endoprothèse C, non traitée après son extraction du tube PVC et de (b) l'endoprothèse A, traitée selon l'invention, 14 jours après son traitement. Les figures 3c et 3d montrent respectivement l'état de surface des endoprothèses non traitée C et traitée A.

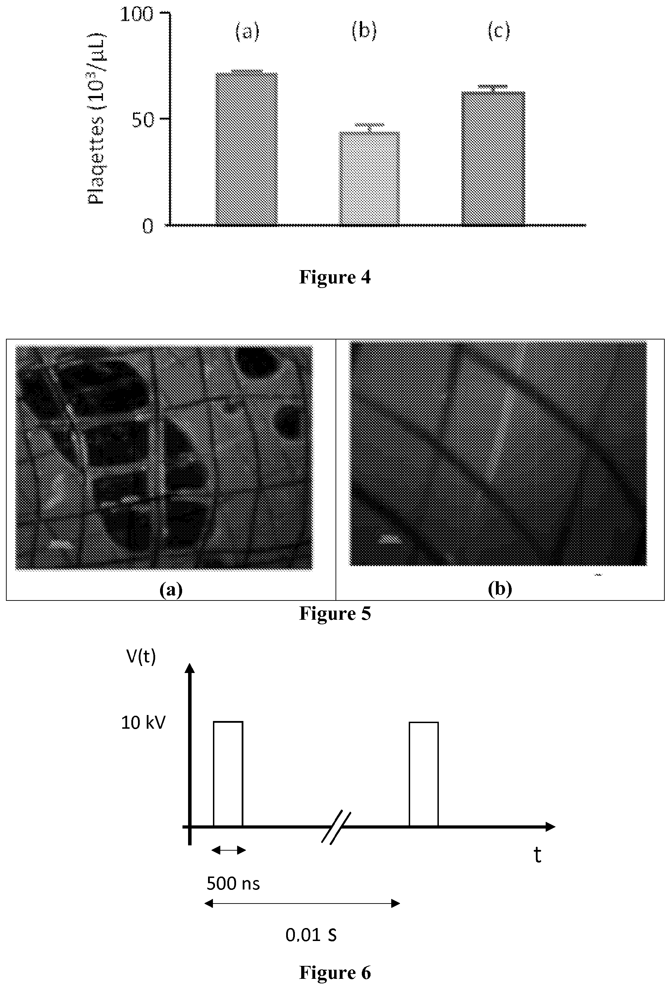

- La figure 4 représente l'impact sur la numération plaquettaire du sang après passage dans la Chandler Loop comprenant une endoprothèse A ou C, tel que décrit dans l'exemple 2. (a) Sang extrait de la tubulure après passage dans la Chandler Loop et en l'absence d'endoprothèse vasculaire ; (b) Sang extrait de la tubulure après passage dans la Chandler Loop et en présence d'une endoprothèse vasculaire C, non-traitée ; (c) Sang extrait de la tubulure après passage dans la Chandler Loop et en présence d'une endoprothèse vasculaire A, selon l'invention.
- La figure 5 représente (a) la surface d'une endoprothèse vasculaire C, non-traitée, et (b) la surface d'une endoprothèse vasculaire A, selon l'invention, observées dans le plan focal d'un microscope épi fluorescent après un marquage des protéines à l'aide d'une sonde NHS Alexa 488.
- La figure 6 représente un exemple de signal électrique (tension en volts en fonction du temps en secondes) pouvant être appliqué au système d'électrodes du dispositif selon l'invention, permettant de générer le champ électrique selon le procédé de l'invention.

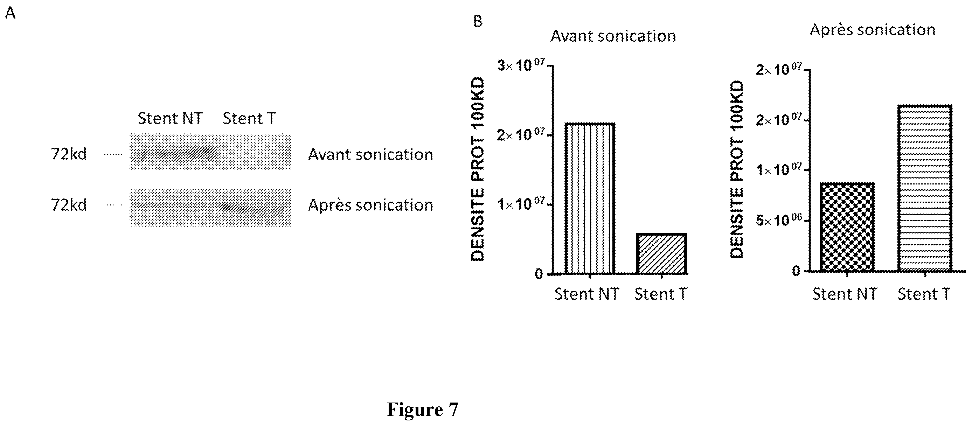

- La figure 7 (A) représente une comparaison des protéines libérées par une endoprothèse A, traitée selon l'invention (T) ou C, non-traité (NT), après traitement chimique et avant / après sonication puis analysées par électrophorèse SDS PAGE. (B) représente la quantification par densitométrie des bandes protéiques séparées par électrophorèse SDS PAGE.

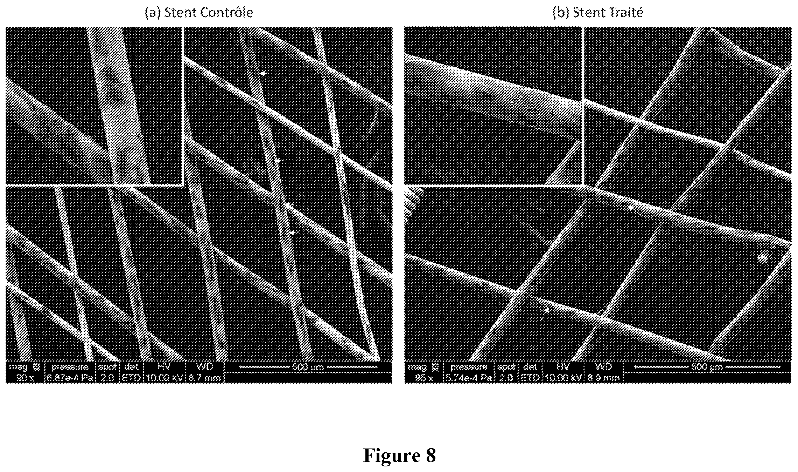

- La figure 8 représente une image de microscopie électronique à balayage montrant la colonisation par les cellules endothéliales humaines (HUVEC) d'une endoprothèse C non-traitée (contrôle) (a) et d'une endoprothèse A, traitée selon l'invention (b), après 5 jours de mise en culture statique. Grossissement de 90x et de 500x pour la fenêtre zoom. (Echelle : 500µm).

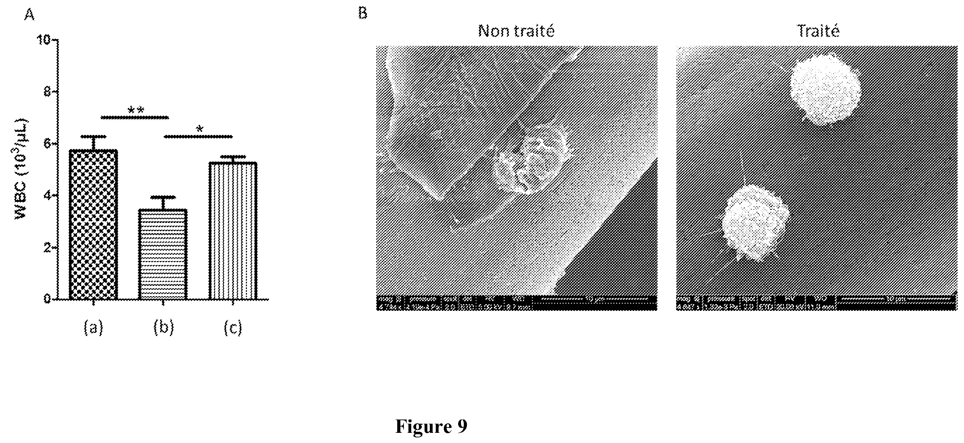

- La figure 9 représente une comparaison de l'adhésion des leucocytes sur une endoprothèse non-traitée et d'une endoprothèse traitée selon l'invention après 1h de rotation d'un sang témoin dans la Chandler Loop. (A) représente la quantité de globules blancs libres dans le sang après la Chandler Loop, (B) est une image de microscopie à balayage illustrant l'aspect des globules blancs interagissant avec la surface d'une endoprothèse C, non-traitée (gauche), ou A, traitée selon l'invention (droite). Grossissement 4700x. (Echelle : 10µm).

### EXEMPLES

### Exemple 1a : Préparation d'une endoprothèse vasculaire selon l'invention (Endoprothèse A)

On insère dans un tube PVC, une première électrode haute tension qui est un fil conducteur recouvert d'un diélectrique (cette électrode est un micro-guide utilisé en neurochirurgie). Le cœur métallique de l'électrode à un diamètre de 170 µm et est recouvert d'une épaisseur de parylène (diélectrique) de 50 µm (cela conduit à un diamètre externe total du micro guide de 270 µm).

On déploie un stent flow diverter nitinol (marque déposée Silk) dans le tube PVC transparent de diamètre interne égal à 3,7 mm et de diamètre externe égal à 6 mm. La paroi interne du tube PVC est héparinisée, c'est-à-dire qu'il est incubé avec de l'héparine, un anticoagulant puissant (elle empêche la formation de fibrine) qui va recouvrir toute la surface du tube PVC et empêcher l'activation des cellules circulantes qui pourrait masquer l'effet du traitement de l'endoprothèse.

La première électrode (micro-guide) est maintenue entre le stent et la paroi interne du tube PVC. La longueur du tube PVC est de 20 cm. La longueur du stent déployé est de l'ordre de 4 cm et une des extrémités du stent se trouve à une distance d'environ 2 cm d'une des extrémités du tube PVC.

A l'autre extrémité du tube PVC, on place un fil conducteur (deuxième électrode de masse) dans le tube PVC. La distance entre le fil conducteur et le stent est d'environ 10 cm. On remplit le tube PVC avec du plasma sanguin (ou PBS contenant de l'albumine) et on clampe les deux extrémités du tube PVC pour éviter que le plasma ne s'écoule.

Les extrémités des fils des première et deuxième électrodes restent accessibles à l'extérieur du tube PVC sur une longueur de plusieurs centimètres.

On relie l'extrémité du fil conducteur à la masse et le cœur métallique de l'électrode haute tension à une alimentation de tension.

On applique pendant 20 minutes des impulsions de tension positive de 10 kV d'amplitude, de 500 ns de durée à une fréquence de 100 Hz.

On obtient l'endoprothèse traitée A.

### Exemple 1b : Préparation d'une endoprothèse vasculaire selon l'invention (Endoprothèse B)

Le stent flow diverter nitinol (marque déposée Silk+ : Blat Extrusion Monmorency, France) est placé dans un tube PVC transparent de diamètre interne égal à 1,6 mm et de diamètre externe égal à 2,4 mm. La longueur du tube PVC est de 20 cm. La longueur du stent déployé est de l'ordre de 2,5 cm et une de ses extrémités est à une distance d'environ 2 cm d'un des bords du tube PVC.

On entoure la surface externe du tube PVC avec une électrode métallique (scotch cuivre) de largeur 1 cm environ. La largeur du scotch recouvre en partie le stent qui est séparé de l'électrode conductrice par l'épaisseur diélectrique du tube PVC. A l'autre extrémité du tube PVC, on place un fil conducteur dans le tube PVC. La distance entre le fil conducteur et le stent est d'environ 8 cm.

On remplit le tube PVC avec du plasma sanguin (ou PBS contenant de l'albumine) et on clampe les deux extrémités du tube PVC pour éviter que le plasma ne s'écoule. L'extrémité du fil conducteur est accessible à l'extérieur du tube PVC sur une longueur de plusieurs cm.

On relie l'extrémité du fil conducteur à la masse et l'électrode externe (le scotch métallique) à une alimentation de tension.

On applique pendant 20 min des impulsions de tension positive de 10 kV d'amplitude, de 500 ns de durée à une fréquence de 100 Hz.

On obtient l'endoprothèse B.

Dans ce qui suit, on appellera endoprothèse C, une endoprothèse de contrôle, non-traitée, ne faisant pas partie de l'invention.

### Exemple 2 : Résultats

Après le traitement, le plasma en contact avec l'endoprothèse A, traitée selon l'invention, de l'exemple 1a est remplacé par du sang humain (il est versé dans la même tubulure en PVC qui a servi pour le traitement). L'ensemble est ensuite placé dans un dispositif Chandler Loop System^{®} (industriedesign, ebo kunze) pour reproduire les conditions rhéologiques de circulation dans un vaisseau sanguin (le tube en PVC sous la forme d'un tore est mis en rotation dans un bain marie à 37°C afin que le sang soit mis en mouvement dans le tube comme il le serait dans une artère). Une endoprothèse C, non traitée, est préparée dans les mêmes conditions opératoires mais sans être soumise au champ électrique. Après 1h de rotation dans la Chandler Loop, les endoprothèses A ou C sont retirées du tube en PVC. Le sang est collecté pour analyses et numération plaquettaire. La Figure 3 (a) montre l'aspect visuel et en microscopie électronique à balayage (MEB) de l'endoprothèse C, non traitée, après son extraction du tube PVC. La surface de l'endoprothèse (voir les flèches blanches) est le siège d'une formation de thrombus plaquettaire et des fils de fibrine sont visibles entre ses mailles. La Figure 3 (b) montre l'aspect de l'endoprothèse A, traitée selon l'invention, 14 jours après son traitement. Aucun thrombus plaquettaire ni filaments de fibrine ne sont observés dans le même modèle. La Figure 3 (b) montre ainsi que les effets perdurent au moins 14 jours après le traitement de l'endoprothèse vasculaire.

Les différents essais réalisés montrent que l'effet perdure au moins jusqu'à 6 mois.

Ainsi, lorsque l'endoprothèse est simplement mise en contact avec le plasma humain (sans l'application du champ électrique), on observe un dépôt épais de protéines comblant les aspérités du nitinol (Figure 3 (c)), alors que le film de protéine(s) induit par l'application du champ électrique est nettement plus fin (Figure 3 (d)). Les écailles de l'alliage nitinol à la surface de l'endoprothèse A sont toujours visibles alors qu'elles ne sont plus visibles sur l'endoprothèse C, non traitée.

La figure 4 montre que l'endoprothèse A, traitée selon l'invention, a un impact sur la numération plaquettaire du sang. Dans les trois cas présentés (a), (b) et (c), le taux de plaquette dans le sang est mesuré après 1h de rotation dans la Chandler Loop. On constate que le taux de plaquettes dans le sang est abaissé dans le cas de l'endoprothèse C, non traitée, suite à leur interaction avec l'endoprothèse (figure 4b) alors qu'en présence d'une endoprothèse A, traitée selon l'invention (figure 4c), on retrouve le niveau de plaquettes libres observé en l'absence d'une endoprothèse vasculaire (figure 4a).

Un marquage des protéines du sang a été réalisé à l'aide d'une sonde (NHS Alexa 488) qui se fixe par liaison covalente sur la partie -NH2 terminales des protéines. Cela permet de visualiser les protéines par microscopie épi fluorescente. La figure 5 montre une comparaison entre une endoprothèse vasculaire non-traitée ayant été en contact avec du sang humain et l'endoprothèse A, traitée selon l'invention, ayant été en contact avec du sang humain pendant une durée de 2h dans la Chandler Loop. L'endoprothèse vasculaire C non-traitée de la figure 5 (a) présente une fluorescence élevée et relativement hétérogène dans le plan focal du microscope à cause de l'adhésion plaquettaire et du réseau de fibrines en formation (thrombose). L'endoprothèse traitée selon l'exemple 1 représenté sur la figure 5 (b) présente une fluorescence homogène dans le plan focal du microscope uniquement sur la surface du stent (épaisseur homogène) indiquant la présence du film d'au moins une protéine présente sur la surface de l'endoprothèse A, traitée selon l'invention.

### Exemple 3 : identification et quantification des protéines déposées sur l'endoprothèse

L'endoprothèse a été soumise à différentes conditions de traitements (non-traité et traité selon l'invention), puis incubée dans 300 µl de tampon de laemmli (4 % SDS, 10 % DTT, 20 % glycérol, 0,004 % bleu bromophénol, 0,125M TRIS HCL ; pH = 6,8) pendant 10 minutes à 90°C puis 1 min sous agitation. Les endoprothèses sont récupérées puis sont à nouveau trempées dans le même volume de tampon de laemmli mais cette fois elles sont soniquées par un cycle d'ultrasons pendant 40 secondes à une fréquence de 40kHz. Un volume de 40µl de la suspension est ensuite analysé par électrophorèse SDS PAGE.

Après migration, les protéines seront révélées pour une coloration à l'Imperial stain (BIORAD) et la zone correspondant à l'albumine (protéine majoritaire observée) est montrée dans la figure 7A

Contrairement à l'endoprothèse non-traitée (NT) l'endoprothèse traitée (T) nécessite une étape de sonication pour que le film de protéine déposé puisse être récupéré et analysé, comme le montre les figures 7A et 7B. Ce résultat démontre que le film protéique déposé lors du traitement de l'endoprothèse traitée est très fortement accroché à la surface de l'endoprothèse ce qui corrèle avec l'observation de la persistance de l'effet du traitement de l'endoprothèse pendant une longue durée (6 mois à 4°C).

### Exemple 4 : test d'endothélialisation en culture cellulaire statique

Après traitement par le champ électrique, l'endoprothèse A, traitée selon l'invention, a été retirée du tube PVC et placée pendant 5 jours dans un milieu de culture contenant des cellules endothéliales humaines (HUVEC) en suspension. Les images obtenues en microscopie électronique à balayage des endoprothèses montrent que le traitement n'empêche pas les cellules endothéliales de coloniser la surface de l'endoprothèse en condition statique. Les tâches noires observées sur la figure 8 (a et b) correspondent aux cellules endothéliales adhérées (dont certaines sont indiquées par des flèches). Les images de la figure 8 montrent clairement que les cellules endothéliales adhérent à la surface et que le taux de couverture à 5 jours est identique pour une endoprothèse C, non-traitée (a) que pour une endoprothèse A, traitée selon l'invention (b).

### Exemple 5 : effet du traitement sur le recrutement des leucocytes

On constate que le traitement de l'endoprothèse selon l'invention a aussi un impact sur le recrutement des leucocytes circulants après 1h de rotation dans la Chandler Loop, comme le montre la figure 9A. Lorsque l'endoprothèse est non traitée, la numération des globules blancs dans le sang après 1 heure de rotation est abaissée, ce qui n'est pas le cas quand l'endoprothèse est traitée. De plus l'observation au microscope électronique à balayage (Figure 9B) montre que les leucocytes sont très étalés sur l'endoprothèse non traitée (signe d'une adhésion forte et d'une activation cellulaire). Au contraire, les rares leucocytes qui interagissent avec l'endoprothèse traitée selon l'invention gardent leur forme arrondie (leur attache à la surface de l'endoprothèse est donc fragile).

Ce résultat démontre un impact bénéfique du traitement de l'endoprothèse sur les processus de recrutement et d'activation des leucocytes, pouvant diminuer l'aspect inflammation généralement observé lors de la pose d'une endoprothèse.

### Listes des références

[1] WO2017/004598

## Revendications

1. Endoprothèse vasculaire, déployée ou non-déployée, dont la surface est recouverte par un film comprenant au moins une protéine d'intérêt ayant été soumise à un champ électrique présentant une structure périodique.

2. Endoprothèse vasculaire selon la revendication précédente, dans laquelle la au moins une protéine d'intérêt est choisie dans le groupe comprenant les protéines du plasma sanguin, de préférence l'albumine, et une macromolécule biologique de synthèse, et leurs mélanges.

3. Endoprothèse vasculaire selon l'une quelconque des revendications précédentes, dans laquelle la concentration en protéine d'intérêt sur la surface de l'endoprothèse est supérieure ou égale à 2 µg/cm².

4. Endoprothèse vasculaire selon l'une quelconque des revendications précédentes, dans laquelle l'endoprothèse vasculaire comprend un ou plusieurs alliages choisis dans le groupe comprenant les alliages acier inoxydable, nickel/titane, tantale, cobalt/chrome, platine/chrome, les alliages comprenant éventuellement du magnésium, et leurs mélanges.

5. Procédé de recouvrement de la surface d'une endoprothèse vasculaire, déployée ou non-déployée, par un film d'au moins une protéine d'intérêt, comprenant les étapes de :
- mise en contact de l'endoprothèse vasculaire et d'une solution aqueuse comprenant au moins une protéine d'intérêt,
- application d'un champ électrique, présentant une structure périodique, généré par un système d'électrodes comprenant au moins une première électrode, un diélectrique et au moins une seconde électrode, ledit diélectrique isolant électriquement l'endoprothèse et la au moins une seconde électrode de la au moins une première électrode, et
- recouvrement de la surface de l'endoprothèse par le film d'au moins une protéine d'intérêt.

6. Procédé selon la revendication précédente, dans lequel la concentration en protéine d'intérêt dans la solution aqueuse est supérieure ou égale à 0,1 mg/ml.

7. Procédé selon l'une quelconque des revendications 5 à 6, dans lequel la protéine est choisie dans le groupe comprenant les protéines du plasma sanguin, de préférence l'albumine, et une macromolécule biologique de synthèse, et leurs mélanges.

8. Procédé selon l'une quelconque des revendications 5 à 7, dans lequel le champ électrique est généré par un signal de tension appliqué au système d'électrodes possédant une amplitude allant de 0,1 kV à 50 kV, de préférence de 5 kV à 40 kV, et de manière encore plus préférée de 10 kV à 30 kV, ayant un rapport cyclique allant de 5.10⁻⁶ à 1 de préférence de 5.10⁻⁶ à 5.10⁻³ et ayant une fréquence allant de 0,1 Hz à 100 kHz, de préférence de 1 Hz à 1 kHz.

9. Procédé selon l'une quelconque des revendications 5 à 8, dans lequel le champ électrique est appliqué pendant une durée supérieure ou égale à 10 secondes, de préférence supérieure ou égale à 5 minutes et de manière encore plus préférée supérieure ou égale à 10 minutes.

10. Dispositif de mise en œuvre du procédé selon l'une quelconque des revendications 5 à 9, comprenant :
- au moins une première électrode,
- au moins une seconde électrode,
- au moins un diélectrique isolant la première électrode de la seconde électrode,
- un élément d'accueil susceptible de contenir l'endoprothèse vasculaire, ledit élément d'accueil ayant une forme adaptée à la forme de l'endoprothèse vasculaire, ledit élément d'accueil étant identique ou différent du diélectrique et,
- une source d'énergie électrique connectée aux électrodes et un régulateur de tension permettant de réguler la tension sur la au moins une première électrode et la au moins une seconde électrode afin de générer un champ électrique présentant une structure périodique.

11. Kit comprenant un dispositif selon la revendication 10, une solution aqueuse comprenant au moins une protéine telle que définie à la revendication 7, et éventuellement, une endoprothèse vasculaire.

12. Utilisation du dispositif ou du kit selon l'une quelconque des revendications 10 à 11 pour le recouvrement d'endoprothèse vasculaire par un film d'au moins une protéine d'intérêt.

## Patentansprüche

1. Gefäßendoprothese, expandiert oder nicht expandiert, deren Oberfläche mit einem Film überzogen ist, der mindestens ein Protein von Interesse enthält, das einem elektrischen Feld mit periodischer Struktur ausgesetzt wurde.

2. Gefäßendoprothese nach dem vorhergehenden Anspruch, wobei mindestens ein interessierendes Protein aus der Gruppe ausgewählt ist, die Plasmaproteine des Blutes, vorzugsweise Albumin, sowie ein synthetisch hergestelltes biologisches Makromolekül und deren Mischungen umfasst.

3. Gefäßendoprothese nach einem der vorhergehenden Ansprüche, wobei die Konzentration des interessierenden Proteins auf der Oberfläche der Endoprothese größer oder gleich 2 µg/cm² ist.

4. Gefäßendoprothese nach einem der vorhergehenden Ansprüche, wobei die Gefäßendoprothese eine oder mehrere Legierungen umfasst, ausgewählt aus der Gruppe bestehend aus Edelstahl, Nickel/Titan, Tantal, Kobalt/Chrom, Platin/Chrom, Legierungen gegebenenfalls mit Magnesium, und deren Mischungen.

5. Verfahren zur Beschichtung der Oberfläche einer Gefäßendoprothese, expandiert oder nicht expandiert, mit einem Film, der mindestens ein interessierendes Protein umfasst, wobei das Verfahren folgende Schritte umfasst:
- Kontaktieren der Gefäßendoprothese mit einer wässrigen Lösung, die mindestens ein interessierendes Protein enthält,
- Anlegen eines elektrischen Feldes mit periodischer Struktur, erzeugt durch ein Elektrodensystem, das mindestens eine erste Elektrode, ein Dielektrikum und mindestens eine zweite Elektrode umfasst, wobei das Dielektrikum die Endoprothese elektrisch von der mindestens eine zweite Elektrode der mindestens eine erste Elektrode isoliert, und
- Beschichtung der Oberfläche der Endoprothese mit dem Film, der mindestens ein interessierendes Protein enthält.

6. Verfahren nach dem vorhergehenden Anspruch, bei dem die Konzentration des interessierenden Proteins in der wässrigen Lösung größer als oder gleich 0,1 mg/ml ist.

7. Verfahren nach einem der Ansprüche 5 bis 6, wobei das Protein ausgewählt ist aus der Gruppe, die Blutplasmaproteine, vorzugsweise Albumin, und ein synthetisches biologisches Makromolekül sowie Mischungen davon umfasst.

8. Verfahren nach einem der Ansprüche 5 bis 7, wobei das elektrische Feld durch ein Spannungssignal erzeugt wird, das an ein Elektrodensystem angelegt wird, das eine Amplitude im Bereich von 0,1 kV bis 50 kV aufweist, vorzugsweise von 5 kV bis 40 kV und besonders bevorzugt von 10 kV bis 30 kV, mit einem Tastverhältnis im Bereich von 5.10⁻⁸ bis 1, vorzugsweise von 5·10⁻⁶ bis 5·10⁻³, und mit einer Frequenz im Bereich von 0,1 Hz bis 100 kHz, vorzugsweise von 1 Hz bis 1 kHz.

9. Verfahren nach einem der Ansprüche 5 bis 8, bei dem das elektrische Feld für eine Dauer von mehr als oder gleich 10 Sekunden, vorzugsweise mehr als oder gleich 5 Minuten und noch bevorzugter mehr als oder gleich 10 Minuten angelegt wird.

10. Vorrichtung zur Durchführung des Verfahrens nach einem der Ansprüche 5 bis 9, umfassend:
- mindestens eine erste Elektrode,
- mindestens eine zweite Elektrode,
- mindestens ein Dielektrikum, das die erste Elektrode von der zweiten Elektrode isoliert,
- ein Aufnahmeelement, das geeignet ist, die Gefäßendoprothese aufzunehmen, wobei das Aufnahmeelement eine an die Form der Gefäßendoprothese angepasste Form aufweist und das Aufnahmeelement identisch mit oder verschieden vom Dielektrikum ist, und
- eine elektrische Energiequelle, die mit den Elektroden verbunden ist, sowie ein Spannungsregler, der die Spannung an mindestens einer ersten Elektrode und an mindestens einer zweiten Elektrode regelt, um ein elektrisches Feld mit periodischer Struktur zu erzeugen.

11. Kit, das eine Vorrichtung gemäß Anspruch 10 umfasst, eine wässrige Lösung, die mindestens ein Protein wie in Anspruch 7 definiert enthält, und gegebenenfalls eine Gefäßendoprothese.

12. Verwendung der Vorrichtung oder des Kits nach einem der Ansprüche 10 bis 11 zur Beschichtung einer Gefäßendoprothese mit einem Film, der mindestens ein interessierendes Protein enthält.

## Claims

1. Vascular stent, deployed or non-deployed, whose surface is coated by a film comprising at least one protein of interest which has been subjected to an electric field having a periodic structure.

2. A vascular stent according to the preceding claim, wherein the at least one protein of interest is selected from the group comprising blood plasma proteins, preferably albumin, and a synthetic biological macromolecule, and mixtures thereof.

3. A vascular stent according to any of the preceding claims in which the concentration of protein of interest on the surface of the stent is greater than or equal to 2 µg/cm².

4. A stent according to any of the preceding claims, wherein the stent comprises one or more alloys selected from the group consisting of stainless steel, nickel/titanium, tantalum, cobalt/chromium, platinum/chromium alloys, alloys optionally including magnesium, and mixtures thereof.

5. A process of coating the surface of a vascular stent, deployed or non-deployed, with a film of at least one protein of interest, comprising the steps of :
- bringing the vascular stent into contact with an aqueous solution comprising at least one protein of interest,
- application of an electric field, having a periodic structure, generated by a system of electrodes comprising at least one first electrode, a dielectric and at least one second electrode, said dielectric electrically isolating the stent and the at least one second electrode from the at least one first electrode, and
- coating of the surface of the stent with the film of at least one protein of interest.

6. A process according to the preceding claim, in which the concentration of protein of interest in the aqueous solution is greater than or equal to 0.1 mg/ml.

7. A process according to any of claims 5 to 6, wherein the protein is selected from the group comprising blood plasma proteins, preferably albumin, and a synthetic biological macromolecule, and mixtures thereof.

8. A process according to any one of claims 5 to 7, wherein the electric field is generated by a voltage signal applied to the system of electrodes having an amplitude ranging from 0.1 kV to 50 kV, preferably from 5 kV to 40 kV, even more preferably from 10 kV to 30 kV, having a duty cycle ranging from 5.10⁻⁸ to 1, preferably from 5.10⁻⁶ to 5.10⁻³, and having a frequency ranging from 0.1 Hz to 100 kHz, preferably from 1 Hz to 1 kHz.

9. A process according to any one of claims 5 to 8, in which the electric field is applied for a duration greater than or equal to 10 seconds, preferably greater than or equal to 5 minutes and even more preferably greater than or equal to 10 minutes.

10. A device for carrying out the process according to any of claims 5 to 9, comprising :
- at least one first electrode,
- at least one second electrode,
- at least one dielectric isolating the first electrode from the second electrode,
- a receiving element capable of containing the vascular stent, said receiving element having a shape adapted to the shape of the vascular stent, said receiving element being identical or different from the dielectric,
- a source of electrical energy connected to the electrodes and a voltage regulator for regulating the voltage on the at least one first electrode and the at least one second electrode in order to generate the electric field having a periodic structure.

11. A kit comprising a device according to claim 10, an aqueous solution comprising at least one protein as defined in claim 7, and optionally a vascular stent.

12. Use of the device or kit according to any one of claims 10 to 11 for coating a vascular stent with a film of at least one protein of interest.
